# EUROPEAN PATENT SPECIFICATION

(11) **EP 3 600 666 B1**
(45) Date of publication and mention of the grant of the patent: **06.01.2021**
(21) Application number: 18723956.1
(22) Date of filing: 28.03.2018
(51) Int. Cl.: B01L 3/00, G01N 1/28, B01D 17/02, A61B 5/15, G01N 33/49

(54) **MICROFLUIDIC SYSTEM**
MIKROFLUIDISCHES SYSTEM
SYSTÈME MICROFLUIDIQUE

(30) Priority: 31.03.2017 PL 42107417
(43) Date of publication of application: 05.02.2020
(73) Proprietor: PZ Cormay S.A., 05-092 Lomianki (PL); Scope Fluidics S.A., 01-796 Warszawa (PL)
(72) Inventor: OSTROWSKI, Miroslaw, 02-136 Warszawa (PL); PRUSAK, Kamil, 28-100 Busko Zdrój (PL); GARSTECKI, Piotr, 01-494 Warszawa (PL); DMITRIEW, Adam, 01-494 Warszawa (PL); TOMALA, Mateusz, 07-310 Ostrów Mazowiecka (PL); SULIMA, Magdalena, 26-713 Kazanów (PL); BANIA, Krystian Józef, 36-200 Brzozów (PL)
(74) Representative: Godlewski, Piotr
(86) International application number: PCT/IB2018/052127
(87) International publication number: WO 2018/178895

(56) References cited:
- WO-A1-2014/049116
- WO-A2-2013/045695
- CN-A- 103 852 576

## Description

The invention relates to a microfluidic system for fluid specimen collection, especially blood, by means of capillary forces, and separation thereof by centrifugation. The invention finds application in medical diagnostics.

Medical blood testing has become a widespread method of detecting a number of various medical conditions. Blood collection from a patient is mostly performed with disposable, sterile blood collection tubes with needles, such as those disclosed, for example, in US2010323437A1, KR20080025050A or JP2000189406A publications. Usually, the tubes are a few centimetres long, and their volumes range from a few hundred microlitres to a few millilitres. It is also possible to collect capillary blood from patient's finger to a tube through a capillary wetted by blood. Then, blood cells are separated from the blood plasma/serum by centrifugation. The vast majority of blood tests are performed on plasma or serum instead of on whole blood because blood cells affect test results.

Centrifugation is a method of choice for plasma/serum extraction from whole blood for clinical assays due to excellent plasma extraction efficiency (with almost 100% purity), high plasma yield, low separation times and suppression of haemolysis. After centrifugation, blood cells are deposited at the bottom of the tube, with the plasma (or serum) above. The blood samples so prepared are placed in vertical stands and analysed in biochemistry analyzers designed to determine various chemicals in blood, including glucose, lipids (e.g., cholesterol, triglycerides), enzymes or ions. A biochemistry analyzer collects a serum (or plasma) sample from a tube, mixes it with appropriately selected reagents in a reaction cell, and by measuring products of chemical reaction, for example photometrically, the concentration of chemicals of interest in the blood sample can be determined.

The process of obtaining the blood serum (or blood plasma) needed for medical tests and subsequent specimen delivery to an analyzer is complex, quite invasive for a patient and requires many operations, including filling a tube, tube centrifugation, and multiple collection of serum (or blood) from the tube for consecutive assays. The existing procedures require performance of a number of operations related to transporting of blood and serum (or plasma) samples between different vessels and/or hydraulic tubing, often involving manual or mechanical operations. Reduction of the number of operations on blood and serum (or plasma) specimens, in particular in the initial stages of the process - i.e., blood collection and separation into light (plasma/serum) and heavy (blood cells) phases (e.g., by centrifugation) would be extremely advantageous, resulting in simplified procedures, minimization of errors and contaminations, and shortening of time from blood collection to analysis. Another desirable technical improvement in this area is a possibly effective protection against remixing of phases after they have been separated and the issue of how easily and quickly the separated light phase can be collected.

Microfluidic analysis techniques have more recently received greater attention as they have the advantage of being able to handle much smaller fluid volumes in the order of microlitres. A so-called "lab-on-a-chip" enables droplets to be generated in microchannels with very small volumes ranging from microlitres through nanolitres down to picolitres. Microfluidic analysis systems are especially of interest in the medical field where fluid analysis is routinely performed. In blood analysis, for example, such systems have the advantage that the volume of the sample required to perform analysis is very small in comparison to standard analysis techniques, thereby reducing discomfort experienced by the patient. Collecting a drop of capillary blood, instead of several millilitres of blood drawn from veins, is easier, requires minimal training, and reduces patient's injuries and trauma, especially in infants, children and the elderly.

Two types of devices based on two mechanisms of capillary blood aspiration, are essentially used in this area: negative pressure in a collection device and capillary forces. Negative pressure can be generated in a rigid and tight device or in a device that is elastically deformable by squeezing and subsequent pressure release after applying the inlet to a blood source. The negative pressure-based solution has, however, disadvantages including the following:
- negative pressure-based devices are expensive in operation;
- too intense blood aspiration can result in haemolysis;
- vacuum present in a vessel can result in collapse of blood vessels in a patient;
- prepared vessel can be unintentionally made untight before blood collection - then it is either generally unsuitable for use or the achieved degree of filling of the vessel with blood is insufficient. For vessels, which are made of rigid material, and especially of glass - it is not possible to detect such lack of tightness organoleptically before use.

Therefore, the devices for capillary blood collection which aspirate blood based on action of capillary forces seem to be the most interesting ones.

A very simple device of this sort is disclosed in US3926521A. The device comprises a tube made of rigid, transparent material, constricting at one end, forming a capillary tube. Blood is aspirated, because a vent hole is provided in the side wall of the tube. With inappropriate positioning of the device, blood can flow out through the hole. After the blood specimen is drawn, a cap made of flexible material is applied on the tube and forms a tight connection around the tube perimeter. Through the capillary tube blood can flow into the cap. The device does not comprise any protection against remixing of phases after separation. Collection of the light phase after the phase separation is difficult and arduous.

Another example of such a device is disclosed in US4024857. The reported device is designed for collection of small quantities of blood (about 100 microlitres) and comprises a cup which is plastic moulded and a top (cap) with a vent hole, with a capillary tube designed for aspiration of a blood specimen into the cup primarily due to the force of gravity. As an option, it comprises also a stopper enabling to tightly close the cup after the blood collection is completed. As the top must be vented to enable air escape from the cup, which is to be replaced by blood - the use of the stopper is necessary in order to tightly close the blood specimen in the cup (e.g., for mixing, transport, storage, etc.).

Improvements to the device described above are known from, for example, US4215700A, where appropriate stoppers are fixed with flexible material straps to the device and radial extensions are used outside the tube, allowing for centrifugation in a centrifuge designed for larger containers, or from US4646753A, where the blood flow between the capillary tube and the cup is improved.

If an agent preventing remixing of phases after their separation by centrifugation is used in the devices described above, it is a thixotropic gel with density and other parameters appropriately selected so as to enable the gel to position between phases. A reference to possible application of such a gel is contained in US4215700A.

Another device is known from EP0545500A1. The device comprises, similarly as those discussed earlier, a sharply ended capillary tube for blood collection. The inside of the capillary tube is filled with foam with specific structure that accelerates blood aspiration. The blood is separated inside the foam. The inventors disclose that the foam plays role of an agent preventing phase remixing and is the only, sufficient means for the purpose, offering in this area an advantage over the gel mentioned above. A detailed analysis of flow mechanisms of blood and blood cells through the foam, as discussed in the above mentioned patent application, casts doubts on the efficiency of the foam effect.

Other such devices where blood collection is caused by capillary forces are known from Polish P-399243 and P-400953 patent applications (as well as from their international equivalents, i.e., PCT/EP2012/069341 and PCT/EP2013/070180, respectively). They are microfluidic systems fabricated in essentially cubicoidal pieces of plastic (so called chips), designed for blood testing, providing capability of blood aspiration (collection) into the system, centrifugation thereof in the system and separation into light (plasma/serum) and heavy (blood cells) fractions, and subsequently pushing out (plasma/serum) from the system, in order to perform required analyses. Therefore, these systems address the above mentioned need of minimizing the blood volume collected for tests, as well as that of minimizing the number of operations performed on a blood specimen - because they combine three basic functions: specimen collection, specimen separation by centrifugation and specimen partitioning/dispensing.

In the course of further work with such systems the inventors of the present solution have found that fabrication of microfluidic systems such as those reported in P-399243 and P-400953 results in necessity to fabricate microfluidic channels very precisely, especially when such system includes a T-type junction that is used for partitioning (dispensing) the specimen by generating microdroplets from it. Additionally, in the case of a layered system design (typically, systems known from P-399243 and P-400953 are fabricated from two plastic layers - upper and lower ones - which comprise components of these systems - channels, holes, etc.) - it is necessary to position the system layers in relation to each other and to assembly them in a very precise manner. In practice, it makes fabrication of such systems considerably more difficult. The systems disclosed in P-399243 and P-400953 lead out specimen portions for tests (microdroplets) via a dedicated microfluidic channel terminated with a hole located at an earlier designated point of the system, wherefrom the microdroplets must be received for further analysis. It means that these systems can cooperate with a dedicated analytical device only, which by its design is adapted to receive specimen portions from an appropriate point in the system, or it is necessary to use an appropriate adapter/interface to transfer such a specimen portion.

Considering high requirements for devices fabricated according to the designs disclosed in P-399243 and P-400953, it turned out that an extremely practical compromise between the need to maximize the number of operations on the specimen to be performed inside a single system and the possibility of its cooperation with existing dispensing devices is to resign from the dispensing function (partition into portions) at a T-junction of the microfluidic system channels and adapt its design so that after specimen separation by centrifugation, the chamber with desired specimen fraction could be accessible with a standard dispensing device (such as pipette or syringe) in the simplest and fastest possible way. In particular, if a microfluidic system has the form of a plastic plate limited from below by the lower plane, and from above - by the upper plane, essentially parallel to the lower plane, as well as limited from the side by the side surface connecting the lower and upper planes, and is normally used in horizontal position, i.e., such one where said upper and lower planes are essentially parallel to horizontal, it is anticipated that the system elements such as microfluidic chambers and channels are comprised in a certain layer of the system and run essentially horizontally, and the chamber comprising, after centrifugation, the light fraction (e.g., plasma) of a (e.g., blood) specimen can be accessed from the top. It should be stressed that the term "layer" as used here (i.e., where system elements such as microfluidic chambers and channels are located) refers to a layer that can be logically separated in a microfluidic system. Such a "layer" does not necessarily correspond to layers, which are physically structural components of the system. In particular, in a preferred embodiment of the present invention, the microfluidic system comprises two layers of material - the upper and the lower one, whereas the microfluidic chambers and channels are so positioned in the system that a part of them is located in the upper layer of the material, a part - in the lower layer, and a part is partitioned between the upper and the lower layer (for example - a part of the first chamber is located in the lower layer, and a part - in the upper layer). In the case referred to here, "normal use" means position, in which the system is placed in the centrifuge rotor in order to separate the collected specimen by centrifugation. Therefore, the system can cooperate with virtually any dispensing device, which is capable of collecting the specimen from a container, for example with a needle. At the same time, integration of specimen collection and separation in a single microfluidic system is preserved, whereby the specimen is continuously comprised in a single and the same system in the process from specimen collection in a patient to specimen dispensing for tests. Following this concept, it was possible to significantly simplify the design of the microfluidic system - in particular to resign from certain elements of the system disclosed in P-400953, wherein said elements were used for specimen dispensing. Moreover, a system so simplified does not include any active (working, moving - and therefore wearable) elements - such as for example valves. It turned out unexpectedly that such a simplification of system design reduced precision requirements for channel fabrication and mutual positioning of system layers in respect to each other, and consequently fundamentally simplified the fabrication process of such a system.

The purpose of the present invention is to provide a microfluidic system for fluid specimen collection, especially blood, and separation thereof by centrifugation, with a design simplified in relation to the prior art and having advantages mentioned above.

For convenience and clarity, the following description makes use of references to the analysis of blood and their fractions, namely plasma/serum (i.e., light fraction) and blood cells (i.e., heavy fraction). Therefore, the names of components of the system according to the invention referring to blood such as "plasma chamber" or "blood cells chamber" shall be understood more widely and generally as "light fraction chamber" and "heavy fraction chamber", respectively, because the use of the present invention is not limited to blood testing, but comprises many other biological fluids like for example urine or cerebrospinal fluid. Therefore, the essence of the invention is the shaping, mutual positioning and interconnection of elements (in particular chambers, channels and junctions) contained in a microfluidic system. In the following description and claims the precise term "first chamber" is interchangeably used with the term "plasma chamber", and the precise term "second chamber" is interchangeably used with the term "blood cells chamber".

A microfluidic system for fluid specimen collection, especially blood, by means of capillary forces, and separation thereof by centrifugation about an axis of rotation of a centrifuge rotor, comprising:
a. the first chamber and the second chamber, wherein these chambers are directly hydraulically interconnected by a constriction;
b. a capillary aspiration channel leading to the first chamber, wherein the inlet of the channel is a hole in the external surface of the system;
c. a vent channel whose first end is a different hole in the external surface of the system;
wherein said elements of the microfluidic system are placed so in this system that after placing the microfluidic system in the centrifuge rotor for centrifugation, the inlet of the capillary aspiration channel and the first end of the vent channel (6) are located closer to the axis of rotation, the first chamber is located further away from the axis of rotation, and the second chamber is furthest away from the axis of rotation;
according to the invention is characterised in that
the second end of the vent channel is directly interconnected with the capillary aspiration channel inside the microfluidic system, in the section between the inlet of the aspiration channel and the first chamber, wherein the first chamber is structurally adapted so that it can be accessed by means of a standard tip for specimen collection, and that it is possible to collect the content from the inside of the first chamber, and
in that the microfluidic system comprises an excess chamber to accommodate the excess fluid, connected to the capillary aspiration channel in the section of the capillary aspiration channel between the first chamber and the second end of the vent channel.

The terms "upper", "lower", "side" as used above, refer to one of the embodiments of the present invention, according to which the system according to the invention, in normal use - after being placed in the centrifuge rotor for separation of collected blood by centrifugation - is oriented horizontally, i.e., so that said upper and lower planes are essentially parallel to horizontal. In no way it does limit, however, the essence of the invention, according to which it is important that the system structure provides that, after specimen separation by centrifugation, the chamber with a desired specimen fraction (i.e., the first chamber) can be accessed by means of a standard dispensing device (such as a pipette or syringe), from a direction essentially deflected from the material layer comprising microfluidic channels and system chambers according to the invention, and preferably - from normal direction to this layer. The above remark concerning the term "layer", in the paragraph preceding the purpose of the invention, applies also in this case.

The excess chamber provides that, after separation of fractions by centrifugation, the plasma in the first chamber is free from blood cells, even if as much blood is collected that the volume of the blood cells is greater than the volume of the second chamber. Therefore, a far-reaching (and hardly achievable in practice) precision with regard to the volume of the collected blood specimen must not be preserved, and a possible excess does not result in contamination of the collected light fraction with the heavy fraction, without necessary adaptation of the point in the system, wherefrom the plasma is collected.

In such a case, the connection of the excess chamber with the capillary aspiration channel is preferably provided by a microfluidic channel running from the capillary aspiration channel to the second chamber, shaped so that after placing the system in the centrifuge rotor for centrifugation, the channel first approaches the axis of rotation, and then moves away from it. Such shaping of the microfluidic channel provides that the specimen is separated between the first and second chamber and the excess chamber during the centrifugation.

Preferably, the excess chamber comprises a constriction in its middle part. The constriction in the middle part of the excess chamber prevents the specimen flow in the opposite direction, i.e. flowing out the specimen from the excess chamber.

Additionally, increasing the flow cross section area at the inlet of said microfluidic channel to the excess chamber prevents the capillary aspiration of plasma to the excess chamber after separation.

Preferably, the connection between the first chamber and the second chamber has the form of a short microfluidic channel.

Preferably, at least a part of the wall of the first chamber is made from a material that can be punctured by a standard tip for specimen collection, for example a needle or an automatic pipette tip, and allows for collection of the content from the inside of the first chamber.

Such shaping of the first chamber provides that the fluid contained in this chamber is accessible for collection through the wall or part of the wall made from a material that can be punctured by any known dispensing device, for example equipped with a needle or pipette tip.

Preferably, the first chamber in the section in plane parallel to the upper plane of the system constricts smoothly towards the second chamber.

Preferably, the first chamber in the section in plane perpendicular to the upper plane of the system and running through the middle of the first chamber and the second chamber constricts smoothly towards the second chamber at least along a certain section.

Such constriction of the first chamber in the horizontal plane (i.e., the parallel one to the upper plane of the system) and in the vertical plane (i.e., the perpendicular plane to it and running through the middle of the first chamber and the second chamber) minimises the risk of remaining of the blood morphotic elements on the surface of the wall of the plasma chamber, and more generally: it minimises the risk of remaining of the blood cells in the plasma chamber due to depositing on the distal wall in respect to the centrifugation axis during the centrifugation.

Preferably, the distance between the second end of the vent channel and the inlet to the fluid excess chamber as measured along the capillary aspiration channel is at least 1.2 mm, preferably about 1.3 mm, and the distance between the inlet to the fluid excess chamber and the first chamber as measured along the capillary aspiration channel is at least 1.2 mm, preferably about 1.3 mm.

Due to appropriate selection of these distances one eliminates the risk of premature (before centrifugation) blood flow from the aspiration channel to the excess chamber (which would result also in collection of a too large amount of blood, and consistently a potentially undesirable presence of blood cells in plasma), as well as the risk of loss of a certain fraction of plasma (after centrifugation) due to displacement thereof by the plasma collecting tip, introduced into the first chamber, from the plasma chamber to the excess chamber. With regard to the distance between the second end of the vent channel and the inlet to the fluid excess chamber as measured along the capillary aspiration channel, the following applies:
- a too short distance would result in a blood flow to the excess chamber during the aspiration (which would be undesirable - during the aspiration the blood should fill the aspiration channel and the vent channel only - it ensures an appropriate volume of the blood; it can enter the excess chamber only during the centrifugation);
- a too long distance means unfounded increase of the system dimensions. In turn, with regard to the distance between the inlet to the fluid excess chamber and the first chamber as measured along the capillary aspiration channel the following applies:
- a too large distance - means unfounded increase of the system dimensions, and
- as far as a too short distance is concerned - the following reasoning is to be carried out: the volume of blood to be separated corresponds to the sum of volumes of the chambers and the constriction between them. If the distance referred to here is zero, then everything above the desired volume flows to the excess chamber during the centrifugation. A pipette tip (or another element for specimen collection) plunged into the first chamber after centrifugation displaces a certain fraction of plasma above the first chamber. If the channel volume along the distance referred to here is at least as large as the volume of the displaced fraction, the displaced plasma does not flow to the excess chamber, but it can be aspirated by the collecting device.

Preferably, the capillary aspiration channel has a round cross section, with diameter from 0.4 mm to 2.5 mm, more preferably from 1.5 mm to 1.9 mm, and most preferably about 1.8 mm.

The dimensions of the capillary aspiration channel, i.e., the characteristic dimension of the cross-section, the cross section area and length, are so selected that they allow for collection of a desired specimen volume by action of capillary forces only.

Preferably, the vent channel has a round cross section, with diameter from 0.3 mm to 2.5 mm, preferably about 0.5 mm.

Preferably, the first end of the vent channel is located in a concavity in the side surface of the microfluidic system.

Placement of the first end of the vent channel in the concavity protects the vent channel against accidental blocking out during the blood aspiration.

Preferably, the system is made of two durably connected plastic layers, bonded ultrasonically, thermally or by laser, preferably ultrasonically,

Preferably, the system is fabricated from a transparent plastic, selected from the group comprising: polystyrene, PMMA, COP, COC, preferably from polystyrene.

Due to the transparent walls that are visible from outside - the vent channel can be used as a marker of correct filling of the system with blood (or other distinctly coloured liquid).

Particularly preferably, the system has the form of a plate, preferably from plastic, limited from below by the lower plane, from above by the upper plane, essentially parallel to the lower plane, and limited from the side by the side surface connecting the lower and upper planes, while the upper plane comprises an access hole leading to the first chamber and placed over the first chamber, allowing the fluid to be aspirated from the first chamber, wherein the access hole is tightly closed before the first use of the system.

Preferably, the access hole leading to the first chamber is sealed with foil, preferably a double-layer one, especially comprising a polystyrene layer from the side of the first chamber and an aluminium layer from the opposite side, wherein said foil can be punctured with a dispensing device, preferably an automatic pipette tip or a needle, while collecting the specimen from the first chamber.

Preferably, the side surface of the system is specially shaped, in particular it comprises undercuts, profiling, cavities or tabs, which facilitate stable fixation of the system in the centrifuge rotor.

Preferably, the elements of the microfluidic system are placed so that after placingthe system in the centrifuge rotor for centrifugation, the axis of rotation is located on the same side of the system as the first end of the vent channel and the inlet of the aspiration channel.

Preferably, the vent channel has transparent walls that are visible from outside of the system and can be used as a marker of correct filling of the system with blood.

Preferably, the system comprises anticoagulants, especially in the form of a coating of the internal surface of the aspiration channel with an anticoagulant agent, preferably heparin.

Preferably, the system comprises a stopper, especially a silicone one, attached to the microfluidic system.

The stopper allows the microfluidic system to be closed and thereby allows an external device (e.g., analyzer) to be protected against contamination with a liquid (blood) from the microfluidic system, and due to attachment to the system - the stopper is protected from being lost.

A device according to the invention has a number of advantages in relation to devices from the prior art. In particular:
- the device allows for blood collection by means of capillary forces only and independently from spatial orientation of the device - in particular with respect to the force of gravity (capillary forces allow for blood aspiration even against the force of gravity);
- the collected blood does not flow out of the system through the valve (vent channel) - independently of spatial orientation of the device (provided that blood collection is stopped after the necessary amount is collected, not later than before the vent channel is filled);
- it has essentially a one-piece structure and can be used both for collection and for separation of blood (as opposed to the device known from US4024857 and similar ones, which must comprise a cup, a cap with a capillary tube, and a stopper);
- it allows for a smooth (i.e., fast and not much labour-intensive) transition from the blood collection step to the phase separation step (it is enough to close the stopper and place it in a centrifuge);
- due to an appropriate chamber design (in a preferred embodiment of the invention) - it reduces the risk of remixing of the light and heavy phases after they have been separated without necessary use of additional technical means (as for example gel or foam);
- it provides an immediate access to the out separated light phase (by centrifugation) (it does not even require to open the stopper - it is sufficient to stick with one move of a pipette, needle or other relatively sharp tip into the plasma chamber);
- it ensures a maximally efficient use of the collected specimen volume, and hence the analyses can be performed while collecting a smaller blood volume.

The present capillary tube differs from that disclosed in P-400953 by positioning of the vent channel with respect to other channels and chambers. In the design known from P-400953, to have the plasma chamber filled with plasma after centrifugation, it was necessary to collect the blood volume at least equal to the sum of the volumes of both chambers, the constriction between them and the long vent channel. In the present capillary tube it is possible to obtain the necessary plasma volume from smaller blood volume, due to a more efficient blood use. This is possible because the vent channel is connected to the aspiration channel. The chambers are not filled during the collection (aspiration). The collected blood specimen can have a volume equal to the sum of the chamber volumes and the constriction between them only.

The invention will now be described in more detail in preferred embodiments, with reference to the accompanying figures, wherein:
Fig. 1 shows an overall view of the microfluidic system according to the invention, in a preferred embodiment;
Fig. 2 shows in detail the design of the lower layer of the system shown in Fig. 1, and
Fig. 3 shows in detail the design of the upper layer of the system shown in Fig. 1.

The following markings were used in the Figures: 1 - first chamber, 2 - second chamber, 3 - constriction between the first and the second chamber, 4 - capillary aspiration channel, 5 - inlet of the capillary aspiration channel, 6 - vent channel, 7 - first end of the vent channel, 8 - second end of the vent channel, 9 - fluid excess chamber, 10 - microfluidic channel providing connection of the fluid excess chamber to the capillary aspiration channel, 11 - constriction in the middle part of the fluid excess chamber, 12 - stopper, 13 - access hole, 14 - side surface, 100 - first (lower) layer of the system, 101 - lower surface, 200 - second (upper) layer of the system, 201 - upper surface.

### Preferred embodiments

### Example of system design

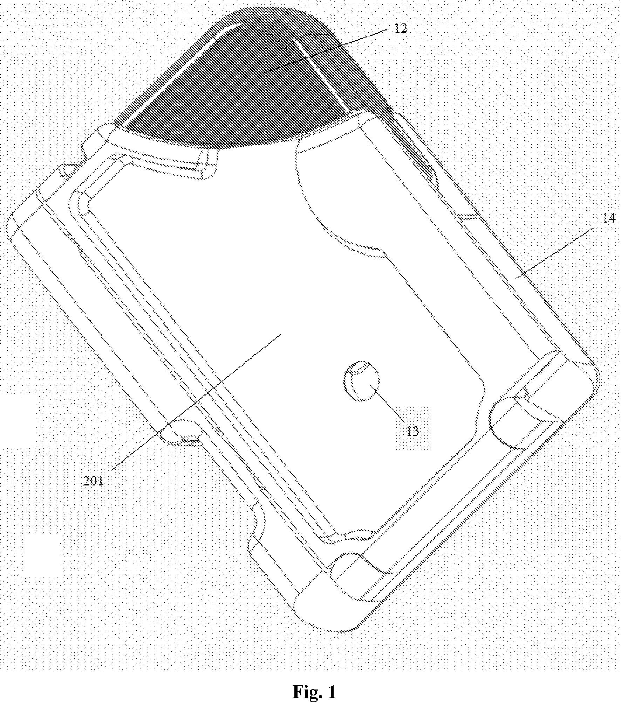

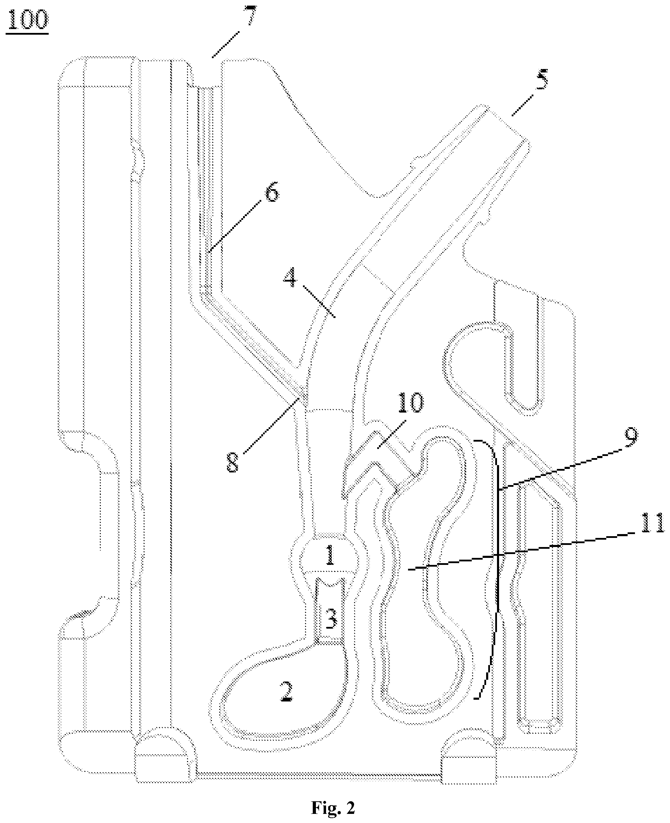

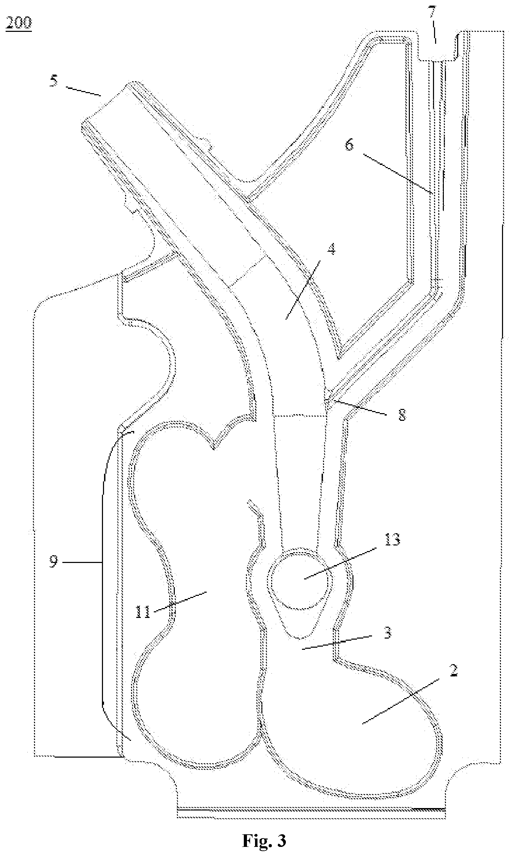

In a preferred embodiment shown in Fig. 1-3, a microfluidic system according to the invention has an essentially planar design, i.e., it has the form of a plate, limited from below by the lower plane 101, from above by the upper plane 201, essentially parallel to the lower plane 101, and limited from the side by the side surface 14 connecting the lower plane 101 and the upper plane 201. The lower plane is not seen in the drawings. It is located behind the element seen in Fig. 2 ("from the bottom of the page"). In the presented embodiment, the lower surface 101 and the upper surface 201 are planes, yet in other embodiments it can be different. Such a form allows for efficient use of the storage space for plates and is convenient in use. A person skilled in the art will notice that it is also possible to spatially arrange channels and chambers in other ways, and consequently, that the system can be formed in a different form, provided that essential features and functions of the system according to the invention are maintained. In addition, the terms used here "upper plane" 201 and "lower plane" 101 do not exclude the presence of grooves, wells and protuberances on the upper and/or lower surface. Such special shape of the upper surface 201 and/or lower surface 101 can result from fabrication process of the system according to the invention or can serve a specific purpose - for example fixing the system in the centrifuge rotor, preventing the system from slipping while holding it in fingers, etc.

Because the microfluidic system is designed for fractionation of specimens contained therein by centrifugation about an axis of rotation of the centrifuge rotor (not shown in figures), the position of the individual elements will be more often specified with reference to the axis of rotation, also known as the axis of centrifugation. The axis runs outside the microfluidic system according to the invention and is perpendicular to the straight line crossing the system essentially in the middle of its height and width, preferably - in the middle of the depth of the aspiration channel 4 and in the middle of the width of the first chamber 1 (light fraction chamber). Preferably, the axis is perpendicular to the plate's lower plane 101 and the upper plane 201. The axis is located on the same side of the system as the first end 7 of the vent channel 6 and the inlet 5 of the aspiration channel 4.

The system according to the invention comprises the capillary aspiration channel 4 used for specimen collection. The inlet 5 of that channel opens towards the outside of the system, so as to enable it for application to a source of a fluid specimen. Although it is essentially possible to position the hole in any (upper 201, side 14, lower 101) system surface, it is preferred that the area of the system external surface surrounding the inlet 5 of the capillary aspiration channel 4 and remaining in contact with the collected specimen is possibly small to minimise the deposition of specimen drops on this surface. In the present embodiment this goal was achieved by forming the initial section of the capillary aspiration channel 4 as a capillary tube, somehow protruding beyond the plate, i.e., located in the indentation in the corner of the plate. The thickness of the tube walls around the inlet 5 of the capillary aspiration channel 4 does not exceed 1.1 mm. Placing the above mentioned indentation in the side surface 14 in the corner of the plate results in withdrawal of the remaining external part of the system surface, which is not designed to contact the specimen, beyond the plane of the inlet 5 of the capillary aspiration channel 4. It reduces the risk of contamination of said external part of the system surface with the specimen.

The system shown in the drawing comprises the aspiration channel 4 with the cross section shaped as a circle. It can be also the shape of another figure, for example rectangle, square, triangle, ellipse, semicircle, etc. An important parameter is the characteristic dimension of the cross section of this channel. Aspiration of a specimen, e.g., blood specimen, takes place by action of the capillary forces only. The dimension must therefore be small enough so that such forces could occur and cause the aspiration channel 4 to fill with blood up to the point, where the vent channel 6 joins the aspiration channel 4. It was assumed that it should not exceed 2.5 mm. The dimensions of the aspiration channel 4 have an effect on rate of specimen aspiration to the system. Increasing the rate is desirable, as it shortens duration of the process. On the other hand, it has been found in the course of testing the present system that a too high rate results in air bubble formation between consecutive drops of collected blood. It is a highly undesirable phenomenon, which can cause that sufficient specimen volume is not collected to the system. Therefore, although the dimension of the cross section of the channel 4 can generally range from 0.4 to 2.5 mm, for a circular cross section the diameter is preferably in the range 1.5 ÷ 1.9 mm, most preferably is from 1.7 to 1.8 mm. In the present embodiment, the circular aspiration channel 4 has a diameter 1.8 mm, which yields the cross sectional area of the channel equal to about 2.5 mm². As follows from the tests on a rectangular channel, in the case of a different shape of the cross-section of the aspiration channel 4, to achieve the optimal effect, the characteristic dimension should be selected so as to preserve the cross sectional area of about 2.5 mm². Such a cross sectional area ensures that the intended specimen volume of about 70 µl can be collected to the system with the assumed plate dimensions (which have an effect on the length of the aspiration channel 4).

The dimensions and shape of the inlet 5 of the aspiration channel 4 in the drawing are identical as the dimensions and shape of the further part of the aspiration channel 4. It is possible, however, that the channel 4 is both constricted and widened in the direction towards the inlet 5. For example, if in a given application, due to reasons discussed above, the formation of the aspiration channel 4 with a very small cross sectional area is preferred, then it may be indicated that the channel is widened funnel-like in the initial section to form the inlet 5 with greater dimensions, facilitating the user to hit with a specimen drop the inlet 5 of the channel 4.

Analogously, the dimensions of the cross section of the aspiration channel can change in its final section, i.e. near the channel's inlet to the first chamber 1 (light fraction chamber), to provide an appropriate connection of the aspiration channel 4 with the first chamber 1. In the system shown in the above drawing, the aspiration channel 4 constricts smoothly towards the light fraction chamber 1, so as to achieve the diameter of the inlet to this chamber of about 1.5 - 1.6 mm. The constriction is one of the factors preventing the withdrawal of the light fraction from the chamber 1 to the aspiration channel 4 after centrifugation.

A necessary condition for the capillary aspiration of the specimen to the aspiration channel 4 to occur is to provide a way to escape from the channel for the air being displaced by the incoming specimen. In the present embodiment, this condition is met by the presence of the vent channel 6. The first end (outlet) 7 of the vent channel 6 opens towards the outside of the system, at a place located not further away from the axis of rotation than the inlet 5 of the aspiration channel 4, preferably at a similar distance from the axis of rotation. Such position of the first end 7 of the vent channel 6 makes sure that the specimen does not get out from it to the outside of the system during the centrifugation. Beyond the centrifugation time, the specimen is kept inside the system by capillary forces. The second end 8 of the vent channel is connected to the aspiration channel 4. When using the system according to the invention, the specimen flows into the system through the inlet 5 of the aspiration channel 4 and fills the aspiration channel 4 up to the point, where it joins the second end 8 of the vent channel 6. Further filling of the aspiration channel 4 by the specimen is not possible, because the further part of the aspiration channel 4 cannot be vented (the access hole 13 remains closed for the time the specimen is being collected to the system). Thus, a further inflow of the specimen is possible only to the vent channel 6. Therefore, the maximum specimen volume that can be collected to the system is equal to the sum of the volume of the vent channel 6 and the volume of the part of the aspiration channel 4, which is located between the inlet 5 and the connection point of the second end 8 of the vent channel 6. Hence, the connection point of the second end 8 of the vent channel 6 to the aspiration channel 4 results from the assumed maximum specimen volume that can be accommodated by the system. The length of the vent channel 6 is conditioned by the fulfilment of the above criteria for positioning of the first 7 and the second 8 ends of the channel 6. The vent channel 6 can essentially have any shape and dimensions of its cross section. Fabrication of a permeable channel 6 with the characteristic dimension below 0.3 mm is hardly achievable in practice.

In the present embodiment of the system according to the invention, at least the final part of the aspiration channel 4 before the connection point of the second end 8 of the vent channel 6, and preferably the entire aspiration channel 4 and the vent channel 6, have transparent walls, so that it is possible to visually check the degree of filling. The filling of the section of the aspiration channel 4 from the inlet 5 to the connection point of the second end 8 of the vent channel 6 means that sufficient specimen volume is collected. In practice, stopping further inflow of the specimen exactly at this moment can be difficult or unrealistic. Even if the inlet 5 of the aspiration channel 4 would be disconnected from the specimen source and the flow would be stopped, for example due to action of capillary forces, a drop of the specimen could remain on the inlet 5 of the aspiration channel 4, outside the system. To prevent this situation, the vent channel 6, apart from the venting function, has also the function of accommodating the excess of the specimen being collected. If the user stops contact the inlet 5 of the aspiration channel 4 with the specimen at the moment when a sufficiently large specimen volume is present inside the system, but a drop, partially still being outside the system, remains on the inlet 5 of the aspiration channel 4, the remaining part of the drop will be able to be aspirated to the system by capillary forces, and the excess volume corresponding to that part - will be accommodated in the vent channel 6. For a given sufficient specimen volume, the volume of the vent channel 6 determines the specimen volume that can be collected to the system. The maximum volume must be reflected in the capacity of the part of the system, to which the sample moves after centrifugation. To prevent undesired growth of the system dimensions, the volume of the vent channel 6 is reduced in the embodiment to about 6 µl, which is achieved with the characteristic dimension of the channel cross section of 0.5 mm.

A consequence of a so small transverse dimension of the vent channel 6 is that it would be easy to cover up accidentally, for example with finger, its outlet 7, located in the external surface of the system, while using the system. It would then impair the basic function of the system, which is to collect the specimen to the aspiration channel 4. In order to minimise the risk, in the present embodiment an indentation was made in the side surface 14 of the system, around the first end (outlet) 7 of the vent channel 6, with dimensions in the plane parallel to the cross section of the vent channel 6, which are several times greater than the dimensions of the channel. It is, however, clear that such indentation is not necessary, and that the same result can be achieved by increasing the transverse dimensions of the entire vent channel or by making sure that the user will not cover up the outlet of this channel during the specimen collection.

The above description concerned the part of the microfluidic system according to the invention, which is used for collection of a fluid specimen. After completion of the collection, the system is centrifuged to separate the specimen into fractions. During the centrifugation, the forces which prevent the specimen flow to further, not vented parts of the system, are overcome. The specimen flows from the vent channel 6 down to the aspiration channel 4, and from there to the separation chambers 1 and 2, moving more and more further away from the axis of rotation.

The second end of the aspiration channel 4 is a point of this channel which is located furthest away from the axis of rotation. It is connected, preferably directly, to the first chamber 1 (light fraction chamber), and through it, through the constriction 3, to the second chamber 2 (heavy fraction chamber). During the centrifugation, the specimen does not only flow from the aspiration channel 4 through the first chamber 1 and the constriction 3 to the second chamber 2, but is also fractionated. The present embodiment was optimized for use for blood fractionation into the cell fraction and plasma. The goal is to obtain plasma which is not contaminated with the blood cells in the first chamber 1. The presence of the blood cells in plasma can adversely affect the results of diagnostic tests carried out on it. In the course of the system testing, there occurred the problem of deposition of the blood cells on the walls of the plasma chamber 1 during the centrifugation. It was found that in order to eliminate the deposition of the blood cells after centrifugation in the first chamber 1, the walls of this chamber from the side of the second chamber 2 must be inclined in a way fulfilling the following condition. The angle between each part of the wall (or any small, planar element approximating given part of the wall) and the straight line running in the middle of the depth of the aspiration channel 4 and in the middle of the width of the first chamber 1 is not greater than 60°. The same applies for the internal surface of the aspiration channel 4.

In order to obtain a blood cells-free plasma in the first chamber 1, also the volumes of the chambers and channels were appropriately selected, taking into account the individual hematocrit variability in the population. In the following, the first chamber 1 and the second chamber 2, i.e., the light fraction chamber and the heavy fraction chamber are referred to as the "separation chambers". The capacity of the second chamber 2 accounts at least for about 60%, preferably about 60% of the specimen volume that is referred to above as "sufficient" because of the anticipated demand for the isolated light fraction, in the case the system comprises an excess chamber, as described below. If the excess chamber 9 or another element with analogous function is absent, the capacity of the second chamber 2 should be at least 60%, preferably about 60% of the maximum specimen volume that can be collected to the system. Illustrative volumes in the present embodiment are given in Table 1 below.

**Table 1. Illustrative volumes of individual modules of the microfluidic system according to the invention**

| | |
|---|---|
| Sufficient specimen volume = capacity of the aspiration channel 4 from the inlet 5 to the connection point of the vent channel 6 | 69.5 µl |
| Excess specimen volume = capacity of the vent channel 6 | 5.8 µl |
| Maximum specimen volume that can be collected to the system | 69 µl + 6 µl = 75 µl |
| Volume of the separation zone (= volume of the plasma zone + volume of the second chamber 2) | 53.09 µl |
| Volume of the plasma zone (= volume of the constriction 3 between the separation chambers + volume of the first chamber 1 + volume of the section of the aspiration channel 4 from the inlet to the first chamber 1 to the connection point of the channel 10 leading to the excess chamber 9) | 21.44 µl |
| Volume of the second chamber 2 (heavy fraction chamber) | 31.65 µl |
| Volume of the excess chamber 9 | 36.5 µl |

The system (as shown in the drawing) is adapted for collection of the light fraction through the access hole 13 formed in the top wall of the first chamber 1. Preferably, the bottom of the first chamber 1 is formed so that directly under the hole 13 it comprises a bottom point which is located lower than all other points of the bottom of the chamber, and into which fluid flows from the entire chamber. In the present embodiment, the bottom of the chamber has the shape of a section of the sphere. It is, however, possible to use any other shape that meets the above conditions and, as a result, allows the entire fluid volume to be collected from the chamber 1 at the lowest point discussed above.

The access hole 13 in the top wall of the first chamber 1 mentioned above must be tightly closed at least until the specimen collection is completed, preferably until the centrifugation is completed. During the specimen collection stage, the only connection of the first chamber 1 to the atmosphere is the connection through the aspiration channel 4 to its inlet 5 and through the aspiration channel 4 and the vent channel 6 to the first end 7 of the vent channel 6. In preferred embodiments - the same happens also during the centrifugation, wherein in this case the inlet 5 of the aspiration channel 4 can be closed, for example, with a stopper 12. The presence of other connections of the inside of the first chamber 1 to the atmosphere in these stages would impair the system operation. Therefore, the hole 13 in the top wall of the first chamber 1 is preferably closed with a material, e.g., a foil or a thin film, which withstands the stresses generated during the centrifugation, but which can be easily punctured or pierced. Preferably, puncture or piercing can be made using the same tool, which is subsequently used for the fluid collection from the first chamber 1, e.g., a pipette tip or a syringe needle. An example of such a material is aluminium foil or laminate.

The separation chambers 1 and 2 are hydraulically interconnected through the constriction 3. Preferably, the connection between the separation chambers has the form of a possibly short section of a microfluidic channel, not shorter, however, than 1.5 mm. This channel may, for example, have a rectangular cross-section, preferably with dimensions ranging from 1 to 1.7 mm, preferably about 1.3 mm. Other shapes of the channel cross section are also possible, while maintaining a comparable cross-sectional area. The presence of the constriction 3 with the given dimensions, between the separation chambers 1 and 2, prevents fraction remixing after centrifugation.

The part of the specimen that after centrifugation was present in the heavy fraction chamber 2, is not collected while the fluid from the light fraction chamber is being collected. As a result, from the point of view of maximizing the efficiency of use of the collected specimen volume, i.e., maximizing the ratio of the volume of the recovered light fraction to the volume of the collected specimen, it is disadvantageous to increase the volume of the second chamber 2 above the necessary minimum, resulting from the content of the heavy fraction in the "sufficient" specimen volume so as to accommodate the heavy fraction contained in the "excess" specimen volume. To maintain the minimum necessary volume of the second chamber 2, while avoiding at the same time shifting of the phase interface to the first chamber 1, the excess chamber 9 was created.

The excess chamber 9 is connected to the aspiration channel 4 via the microfluidic channel 10, between the connection point of the vent channel 6 and the second end of the aspiration channel 4 (i.e., the inlet to the light fraction chamber 1). The microfluidic channel 10, leading from the aspiration channel 4 to the excess chamber 9, initially approaches the axis of centrifugation, preferably running at an angle of no more than about 60 degrees to the direction of centripetal force, coinciding with the axis of symmetry of this section of the aspiration channel 4. Such an angle guarantees that during the centrifugation this channel will be filled with the specimen only after the separation chambers are filled. (For technological reasons regarding the bonding of the system layers 100 and 200, the angle should not be less than 40 degree.) Then, the channel turns, preferably sharply, to move along the further section, from the bend to the excess chamber 9, further away from the axis of rotation. Thanks to this, the excess specimen falls into the excess chamber 9 due to centrifugation. At the connection point of the microfluidic channel 10 to the excess chamber 9, the cross sectional area increases suddenly, i.e., the chamber 9 is significantly wider than the channel 10. This stops the unwanted capillary aspiration of the light fraction from the first chamber 1 to the excess chamber 9. The excess chamber 9 is located essentially further away from the axis of rotation than the microfluidic channel 10. At a point located between their ends: the least and the most distant from the axis of rotation, the chamber 9 constricts, preferably by about 50÷80%, e.g., from 10 to 2-5 mm. During the centrifugation, the possible specimen excess flows near the end of the chamber furthest away from the axis of rotation. After the end of centrifugation, said constriction stops the fluid in this part of the chamber 9 and prevents the flow thereof back to the part located nearer the axis of rotation, which could cause undesirable mixing of the specimen excess with the light fraction in the first chamber 1.

The length of the aspiration channel 4 between the connection point of the vent channel 6 and the connection point of the microfluidic channel 10 leading to the excess chamber 9 is preferably not less than 1.2 mm, preferably about 2 mm. This prevents accidental, undesired flows between modules. Also the length of the section of the aspiration channel 4 between the connection point of the microfluidic channel 10 leading to the excess chamber 9 and the second end of the aspiration channel 4, i.e., the inlet to the first chamber 1, is preferably not less than 1.2 mm, preferably about 2 mm. The length of the latter section may depend, generally, on its cross-sectional area so as to obtain the necessary volume. This section can accommodate the volume of light fraction displaced from the first chamber 1 by, for example, a pipette tip driven into it. The accommodation of the volume in this section means that it does not move irrevocably to the excess chamber 9. After an equivalent light fraction volume is aspirated from the first chamber 1, the displaced volume may flow back to the first chamber 1 and be collected by a dispensing device.

In the present embodiment the system is fabricated from polystyrene. However, it is possible to use any other material whose contact angle formed with a given specimen is such that the specimen can be collected to the aspiration channel 4, fabricated from the same material, by action of capillary forces only, while at the same time the transparency of the channel walls allows for checking visually the degree of the channel filling. For example, the system can be fabricated from another plastic, e.g., PMMA, COP, COC.

The system is fabricated with the injection moulding method. A person skilled in the art will notice that it is possible to use any other method that enables fabricating channels and chambers with given properties, for example milling.

The exemplary system consists of two layers of injection moulded polystyrene, shown in the drawings above, bonded ultrasonically (thermal or laser bonding, etc., is also possible), and a double-layer laminate, wherein the lower layer is polystyrene, and the upper layer is aluminium one, thermally bonded around the hole 13 in the upper polystyrene layer and forming the top wall of the first chamber 1 that can be punctured by a tip of a pipette or another standard dispensing device.

In the present embodiment, the blood collection system contains an anticoagulant, for example in the form of a coating of the internal surface of the aspiration channel 4, for example with heparin.

Preferably, the inlet 5 of the aspiration channel 4 can be closed for the time of centrifugation with a stopper 12. The stopper 12 can be fabricated, for example, from silicone or another elastomer. Preferably, the stopper 12 is permanently attached to the microfluidic system with an elastic strap so as to prevent it from being lost.

### Example of using the system according to its purpose

About 55 µl blood (≥ 54.5 mg) each was collected to 10 microfluidic systems according to the invention by applying the inlet 5 of the aspiration channel 4 to the blood drop. The weight of the blood collected to a given system was determined by subtracting the weight of specific system before the specimen collection from its weight after the specimen collection (Table 2). Lithium heparin was used as an anticoagulant. The inlet 5 of the aspiration channel 4 was closed with a silicone stopper 12. The system was placed in a centrifuge rotor in horizontal position, with upper surface up, and the inlet 5 of the aspiration channel 4 and first end 7 of the vent channel 6 towards the axis of rotation. It was centrifuged for 3 minutes at 6000 rotations per minute (rpm). Then, the foil closing the access hole 13 was punctured with a disposable automatic pipette tip. The tip was inserted vertically through the hole 13 to the first chamber 1 so as to collect fluid from the lowest point of the bottom of the chamber. Then, with an ADP Tecan pump, connected to the tip and appropriately programmed, 15 µl of plasma was collected each time. The actual volume of the plasma collected from the system was calculated as the quotient of the mass difference of a given system - after centrifugation and before plasma collection - and the plasma density, equal to 1.027 g/cm³ (Table 2).

**Table 2**

| Specimen no. | System weight before the specimen collection [mg] | System weight after the specimen collection [mg] | Weight of the collected blood [mg] | Weight of the system with the specimen after centrifugation [mg] | Weight of the system with the specimen after plasma collection [mg] | Weight of the collected plasma [mg] | Volume of the collected plasma [µl] |
|---|---|---|---|---|---|---|---|
| 1 | 4206.02 | 4262.48 | 56.46 | 4261.66 | 4245.47 | 16.19 | 15.76 |
| 2 | 4241.19 | 4301.01 | 59.82 | 4299.75 | 4283.76 | 15.99 | 15.57 |
| 3 | 4209.85 | 4268.44 | 58.59 | 4267.50 | 4251.47 | 16.03 | 15.61 |
| 4 | 4210.42 | 4268.95 | 58.53 | 4268.10 | 4251.19 | 16.91 | 16.47 |
| 5 | 4218.71 | 4277.27 | 58.56 | 4276.50 | 4260.24 | 16.26 | 15.83 |
| 6 | 4178.92 | 4237.02 | 58.10 | 4236.08 | 4219.92 | 16.16 | 15.74 |
| 7 | 4185.10 | 4239.78 | 54.68 | 4238.88 | 4223.14 | 15.74 | 15.33 |
| 8 | 4193.04 | 4252.09 | 59.05 | 4251.23 | 4235.20 | 16.03 | 15.61 |
| 9 | 4237.55 | 4296.1 | 58.55 | 4295.33 | 4279.33 | 16.00 | 15.58 |
| 10 | 4175.78 | 4232.97 | 57.19 | 4232.03 | 4216.20 | 15.83 | 15.41 |

## Claims

1. A microfluidic system for fluid specimen collection, especially blood, by means of capillary forces, and separation thereof by centrifugation about an axis of rotation of a centrifuge rotor, comprising:
a. the first chamber (1) and the second chamber (2), wherein these chambers are directly hydraulically interconnected by a constriction (3);
b. a capillary aspiration channel (4) leading to the first chamber (1), wherein the inlet (5) of the channel is a hole in the external surface of the system;
c. a vent channel (6) whose first end (7) is a different hole in the external surface of the system;
wherein said elements of the microfluidic system are placed so in this system that after placing the microfluidic system in the centrifuge rotor for centrifugation, the inlet of the capillary aspiration channel (5) and the first end (7) of the vent channel (6) are located closer to the axis of rotation, the first chamber (1) is located further away from the axis of rotation, and the second chamber (2) is furthest away from the axis of rotation;
**characterised in that**
the second end (8) of the vent channel (6) is directly interconnected with the capillary aspiration channel (4) inside the microfluidic system, in the section between the inlet of the aspiration channel (5) and the first chamber (1), wherein the first chamber (1) is structurally adapted so that it can be accessed by means of a standard tip for specimen collection, and that it is possible to collect the content from the inside of the first chamber (1), and
**in that** the microfluidic system comprises an excess chamber (9) to accommodate the excess fluid, connected to the capillary aspiration channel in the section of the capillary aspiration channel (4) between the first chamber (1) and the second end (8) of the vent channel (6).

2. The system according to claim 1, **characterised in that** the connection of the excess chamber (9) to the capillary aspiration channel (4) is provided by a microfluidic channel (10) running from the capillary aspiration channel (4) to the second chamber (2), shaped so that after placing the system in the centrifuge rotor for centrifugation, the microfluidic channel (10) first approaches the axis of rotation, and then moves away from it.

3. The system according to claim 1 or 2, **characterised in that** the fluid excess chamber (9) comprises a constriction (11) in its middle part.

4. The system according to any preceding claim 1-3, **characterised in that** the constriction (3) between the first chamber (1) and the second chamber (2) has the form of a short microfluidic channel.

5. The system according to any preceding claim 1-4, **characterised in that** at least a part of the wall of the first chamber (1) is made from a material that can be punctured by a standard tip for specimen collection and allows for collection of the content from the inside of the first chamber (1).

6. The system according to any preceding claim 1 - 5, **characterised in that** the first chamber (1) in the section in plane parallel to the upper plane (201) of the system constricts smoothly towards the second chamber (2).

7. The system according to any preceding claim 1 - 6, **characterised in that** the first chamber (1) in the section in plane perpendicular to the upper plane (201) of the system and running through the middle of the first chamber (1) and the second chamber (2) constricts smoothly towards the second chamber at least along a certain section.

8. The system according to any preceding claim 1 - 7, **characterised in that** the distance between the second end (8) of the vent channel (6) and the inlet to the fluid excess chamber (9) as measured along the capillary aspiration channel (4) is at least 1.2 mm, preferably about 1.3 mm, and the distance between the inlet to the fluid excess chamber (9) and the first chamber (1) as measured along the capillary aspiration channel (4) is at least 1.2 mm, preferably about 1.3 mm.

9. The system according to any preceding claim 1 - 8, **characterised in that** the capillary aspiration channel (4) has a round cross section, with diameter from 0.4 mm to 2.5 mm, more preferably from 1.5 mm to 1.9 mm, and most preferably about 1.8 mm.

10. The system according to any preceding claim 1 - 9, **characterised in that** the vent channel (6) has a round cross section, with diameter from 0.3 mm to 2.5 mm, preferably about 0.5 mm.

11. The system according to any preceding claim 1 - 10, **characterised in that** the first end (7) of the vent channel (6) is located in a concavity in the side surface (14) of the microfluidic system.

12. The system according to any preceding claim 1 - 11, **characterised in that** it is made of two durably connected plastic layers, bonded ultrasonically, thermally or by laser, preferably ultrasonically.

13. The system according to any preceding claim 1 - 12, **characterised in that** it is fabricated from a transparent plastic, selected from the group comprising: polystyrene, PMMA, COP, COC, preferably from polystyrene.

14. The system according to any preceding claim 1-13, **characterised in that** it has the form of a plate, limited from below by the lower plane (101), from above by the upper plane (201), essentially parallel to the lower plane (101), and limited from the side by the side surface (14) connecting the lower plane (101) and the upper plane (201) planes, while the upper plane (201) comprises an access hole (13) leading to the first chamber (1) and placed over the first chamber (1), allowing the fluid to be aspirated from the first chamber (1), wherein the access hole is tightly closed before the first use of the system.

15. The system according to claim 14, **characterised in that** the access hole (13) is sealed with foil, preferably a double-layer one, especially comprising a polystyrene layer from the side of the first chamber (1) and an aluminium layer from the opposite side, wherein said foil can be punctured with a dispensing device, preferably an automatic pipette tip or a needle, while collecting the specimen from the first chamber (1).

16. The system according to any preceding claim 1-15, **characterised in that** side surface (14) is specially shaped, in particular it comprises undercuts, profiling, cavities or tabs, which facilitate stable fixation of the system in the centrifuge rotor.

17. The system according to any preceding claim 1 - 16, **characterised in that** the elements of the microfluidic system are placed so in this system that after placing the system in the centrifuge rotor for centrifugation, the axis of rotation is located on the same side of the system as the first end (7) of the vent channel (6) and the inlet (5) of the aspiration channel (4).

18. The system according to any preceding claim 1 - 17, **characterised in that** the vent channel (6) has transparent walls that are visible from outside of the system and can be used as a marker of correct filling of the system with blood

19. The system according to any preceding claim 1 - 18, **characterised in that** it comprises anticoagulants, preferably in the form of a coating of the internal surface of the aspiration channel (4) with an anticoagulant agent, preferably heparin

20. The system according to any preceding claim 1 - 19, **characterised in that** it comprises a stopper (12), especially a silicone one, attached to the microfluidic system.

## Patentansprüche

1. Ein Mikrofluidik-System zum Sammeln von Flüssigkeitsproben, insbesondere Blut, mithilfe der Kapillarkraft und deren Trennung durch Zentrifugalkraft, um eine Drehachse des Zentrifugenrotors, umfassend:
a. die erste Kammer (1) und die zweite Kammer (2), wobei diese Kammern durch eine Verengung (3) direkt hydraulisch miteinander gekoppelt sind;
b. einen kapillaren Ansaugkanal (4), der zu der ersten Kammer (1) führt, wobei der Eintritt (5) des Kanals als eine Öffnung an der Außenfläche des Systems herausgebildet ist;
c. einen Entlüftungskanal (6), dessen ein Ende (7) als eine weitere Öffnung an der Außenfläche des Systems herausgebildet ist;
wobei die oben genannten Elemente des Mikrofluidik-Systems so in diesem System angeordnet sind, dass nach dem Einsetzen dieses Mikrofluidik-Systems in den Zentrifugenrotor zwecks Trennung der Eintritt des kapillaren Ansaugkanals (5) und das erste Ende (7) des Entlüftungskanals (6) näher an der Rotationsachse angeordnet sind, die erste Kammer (1) weiter von der Rotationsachse entfernt und die zweite Kammer (2) am weitesten von der Rotationsachse entfernt ist;
**dadurch gekennzeichnet, dass**
das zweite Ende (8) des Entlüftungskanals (6) direkt mit dem kapillaren Ansaugkanal (4) im Mikrofluidik-Systems in dem Abschnitt zwischen dem Eintritt des Ansaugkanals (5) und der ersten Kammer (1) verbunden ist, wobei die erste Kammer (1) strukturell so angepasst ist, dass sie mit einer Standardspitze zur Probenentnahme bedient werden kann und die Entnahme des Inhalts aus der ersten Kammer (1) ermöglicht, und
so, dass das Mikrofluidik-System eine Überschusskammer (9) zur Aufnahme der überschüssigen Flüssigkeit umfasst, die an den kapillaren Ansaugkanal in dem Abschnitt des kapillaren Ansaugkanals (4) zwischen der ersten Kammer (1) und dem zweiten Ende (8) des Entlüftungskanals (6) angeschlossen ist.

2. Ein System nach Anspruch 1, **dadurch gekennzeichnet, dass** die Verbindung zwischen der Überschusskammer (9) und dem kapillaren Ansaugkanal (4) durch einen Mikrofluidik-Kanal (10) hergestellt wird, der vom kapillaren Ansaugkanal (4) zur zweiten Kammer (2) verläuft und so geformt ist, dass sich der Mikrofluidik-Kanal (10) nach dem Einsetzen des Systems in einen Zentrifugenrotor zwecks Trennung zunächst der Drehachse nähert und sich dann von ihr entfernt.

3. Ein System nach Anspruch 1 oder 2, **dadurch gekennzeichnet, dass** die Überschusskammer (9) in ihrem mittleren Teil eine Verengung (11) aufweist.

4. Ein System nach einem der vorhergehenden Ansprüche 1-3, **dadurch gekennzeichnet, dass** die Verengung (3) zwischen der ersten Kammer (1) und der zweiten Kammer (2) die Form eines kurzen Mikrofluidik-Kanals aufweist.

5. Ein System nach einem der vorhergehenden Ansprüche 1-4, **dadurch gekennzeichnet, dass** mindestens ein Teil der Wand der ersten Kammer (1) aus einem Material ausgeführt ist, das mit einer Standardspitze zur Probenentnahme durchstochen werden kann und die Entnahme des Inhalts aus der ersten Kammer (1) ermöglicht.

6. Ein System nach jedem der vorhergehenden Ansprüche 1-5, **dadurch gekennzeichnet, dass** sich die erste Kammer (1) in ihrem Querschnitt in einer zur oberen Ebene (201) des Systems parallelen Ebene in die Richtung der zweiten Kammer (2) allmählich verengt.

7. Ein System nach einem der vorhergehenden Ansprüche 1-6, **dadurch gekennzeichnet, dass** sich die erste Kammer (1) in ihrem Querschnitt in einer zur oberen Ebene (201) des Systems senkrechten Ebene und durch die Mitte der ersten Kammer (1) verlaufend, sowie die zweite Kammer (2) sich zumindest entlang eines bestimmten Abschnitts allmählich in die Richtung der zweiten Kammer verengt.

8. Ein System nach einem der vorhergehenden Ansprüche 1-7, **dadurch gekennzeichnet, dass** der Abstand zwischen dem zweiten Ende (8) des Entlüftungskanals (6) und dem Eintritt der Überschusskammer (9), gemessen entlang des kapillaren Ansaugkanals (4), mindestens 1,2 mm, vorzugsweise etwa 1,3 mm, und der Abstand zwischen dem Eintritt der Überschusskammer (9) und der ersten Kammer (1), gemessen entlang des kapillaren Ansaugkanals (4), mindestens 1,2 mm, vorzugsweise etwa 1,3 mm, beträgt.

9. Ein System nach einem der vorhergehenden Ansprüche 1-8, **dadurch gekennzeichnet, dass** der kapillare Ansaugkanal (4) einen runden Querschnitt mit einem Durchmesser von 0,4 mm bis 2,5 mm, bevorzugt von 1,5 mm bis 1,9 mm und optimal von etwa 1,8 mm aufweist.

10. Ein System nach einem der vorhergehenden Ansprüche 1-9, **dadurch gekennzeichnet, dass** der Entlüftungskanal (6) einen runden Querschnitt mit einem Durchmesser von 0,3 mm bis 2,5 mm, vorzugsweise etwa 0,5 mm, aufweist.

11. Ein System nach einem der vorhergehenden Ansprüche 1-10, **dadurch gekennzeichnet, dass** das erste Ende (7) des Entlüftungskanals (6) in einer Wölbung an der Seitenfläche (14) des Mikrofluidik-Systems angeordnet ist.

12. Ein System nach einem der vorhergehenden Ansprüche 1-11, **dadurch gekennzeichnet, dass** dieses zwei dauerhaft verbundenen Kunststoffschichten enthält, die durch Ultraschall, thermisch oder mit Laser, aber vorzugsweise durch Ultraschall, miteinander gebunden sind.

13. Ein System nach einem der vorhergehenden Ansprüche 1-12, **dadurch gekennzeichnet, dass** dieses aus einem transparenten Kunststoff hergestellt ist, ausgewählt aus der Gruppe enthaltend: Polystyrol, PMMA, COP, COC, vorzugsweise aus Polystyrol.

14. Ein System nach einem der vorhergehenden Ansprüche 1-13, **dadurch gekennzeichnet, dass** dieses die Form einer Platte hat, die von unten durch die untere Ebene (101), von oben durch die obere Ebene (201), im wesentlichen parallel zur unteren Ebene (101), und von der Seite durch die seitliche Ebene (14) begrenzt ist, die die untere Ebene (101) und die obere Ebene (201) miteinander verbindet, während die obere Ebene (201) eine Eintrittsöffnung (13) aufweist, die zu der ersten Kammer (1) führt und über der ersten Kammer (1) angeordnet ist, wodurch die Flüssigkeit aus der ersten Kammer (1) aufgenommen werden kann, wobei die Eintrittsöffnung vor der ersten Verwendung des Systems dicht verschlossen wird.

15. Ein System nach Anspruch 14, **dadurch gekennzeichnet, dass** die Eintrittsöffnung (13) mit einer Folie, vorzugsweise einer doppellagigen, verschlossen ist, die insbesondere einer Polystyrol-Schicht an der Seite der ersten Kammer (1) und einer Aluminium-Schicht an der gegenüberliegenden Seite enthält, wobei die Folie mit einer Spendervorrichtung, vorzugsweise einer automatischen Pipettenspitze oder einer Nadel, durchstochen werden kann, während die Probe aus der ersten Kammer (1) entnommen wird.

16. Ein System nach einem der vorstehenden Ansprüche 1-15, **dadurch gekennzeichnet, dass** die Seitenfläche (14) speziell geformt ist, insbesondere umfasst diese Hinterschneidungen, Profilierungen, Hohlräume oder Laschen, die eine stabile Fixierung des Systems im Zentrifugenrotor ermöglichen.

17. Ein System nach einem der vorstehenden Ansprüche 1-16, **dadurch gekennzeichnet, dass** die Elemente des Mikrofluidik-Systems so angeordnet sind, dass nach dem Einsetzen des Systems im Zentrifugenrotor zwecks Trennung die Rotationsachse auf derselben Seite des Systems liegt wie das erste Ende (7) des Entlüftungskanals (6) und der Einlauf (5) des Ansaugkanals (4).

18. Ein System nach einem der vorhergehenden Ansprüche 1-17, **dadurch gekennzeichnet, dass** der Entlüftungskanal (6) transparente Wände aufweist, die von außerhalb des Systems sichtbar sind und zur Prüfung der korrekten Füllung des Systems mit Blut dienen können.

19. Ein System nach einem der vorhergehenden Ansprüche 1-18, **dadurch gekennzeichnet, dass** es Antikoagulantien, vorzugsweise in Form einer Beschichtung der inneren Oberfläche des Ansaugkanals (4) mit einem gerinnungshemmenden Mittel, vorzugsweise Heparin, enthält.

20. Ein System nach einem der vorhergehenden Ansprüche 1-19, **dadurch gekennzeichnet, dass** es einen Stopfen (12), insbesondere aus Silikon, umfasst, der am Mikrofluidik-System befestigt ist.

## Revendications

1. Système microfluidique pour la collecte d'échantillons de fluide, en particulier du sang, au moyen de forces capillaires, et leur séparation par centrifugation autour d'un axe de rotation d'un rotor de centrifugeuse, comprenant :
a. la première chambre (1) et la deuxième chambre (2), lesdites chambres étant directement interconnectées hydrauliquement par un étranglement (3);
b. un canal d'aspiration capillaire (4) menant à la première chambre (1), où l'entrée (5) du canal est un trou dans la surface externe du système ;
c. un canal d'évent (6) dont la première extrémité (7) est un trou différent dans la surface externe du système;
dans lequel lesdits éléments du système microfluidique sont placés de telle sorte dans ce système qu'après avoir placé le système microfluidique dans le rotor de centrifugation pour la centrifugation, l'entrée du canal d'aspiration capillaire (5) et la première extrémité (7) du canal d'évent (6) sont situées plus près de l'axe de rotation, la première chambre (1) est située plus loin de l'axe de rotation, et la seconde chambre (2) est la plus éloignée de l'axe de rotation ;
**caractérisé en ce que**
la deuxième extrémité (8) du canal d'évent (6) est directement interconnectée avec le canal d'aspiration capillaire (4) à l'intérieur du système microfluidique, dans la section entre l'entrée du canal d'aspiration (5) et la première chambre (1), la première chambre (1) étant structurellement adaptée de manière à pouvoir y accéder au moyen d'une pointe standard pour la collecte d'échantillons, et **en ce qu'**il est possible de collecter le contenu de l'intérieur de la première chambre (1), et
**en ce que** le système microfluidique comprend une chambre d'excédent (9) pour recevoir l'excès de fluide, connectée au canal d'aspiration capillaire dans la section du canal d'aspiration capillaire (4) entre la première chambre (1) et la seconde extrémité (8) du canal d'évent (6).

2. Système selon la revendication 1, **caractérisé en ce que** la connexion de la chambre en excès (9) au canal d'aspiration capillaire (4) est assurée par un canal microfluidique (10) allant du canal d'aspiration capillaire (4) à la deuxième chambre (2), agencé de telle sorte qu'après avoir placé le système dans le rotor de centrifugation pour la centrifugation, le canal micro fluidique (10) s'approche d'abord de l'axe de rotation, puis s'en éloigne.

3. Système selon la revendication 1 ou 2, **caractérisé en ce que** la chambre d'excédent de fluide (9) comprend un étranglement (11) dans sa partie centrale.

4. Système selon l'une quelconque des revendications 1 à 3, **caractérisé en ce que** l'étranglement (3) entre la première chambre (1) et la seconde chambre (2) a la forme d'un court canal micro fluidique.

5. Système selon l'une quelconque des revendications 1 à 4, **caractérisé en ce qu'**au moins une partie de la paroi de la première chambre (1) est constituée d'un matériau qui peut être perforé par une pointe standard pour la collecte d'échantillons et permet de collecter le contenu de l'intérieur de la première chambre (1).

6. Système selon l'une quelconque des revendications 1 à 5, **caractérisé en ce que** la première chambre (1) dans la section dans le plan parallèle au plan supérieur (201) du système se resserre doucement vers la deuxième chambre (2).

7. Système selon l'une quelconque des revendications 1 à 6, **caractérisé en ce que** la première chambre (1) dans la section dans le plan perpendiculaire au plan supérieur (201) du système et traversant le centre de la première chambre (1) et la seconde chambre (2) se resserre doucement vers la seconde chambre au moins le long d'une certaine section.

8. Système selon l'une quelconque des revendications 1 à 7, **caractérisé en ce que** la distance entre la deuxième extrémité (8) du canal d'évent (6) et l'entrée de la chambre d'excédent de fluide (9) mesurée le long du canal d'aspiration capillaire (4) est d'au moins 1,2 mm, de préférence d'environ 1,3 mm, et la distance entre l'entrée de la chambre d'excédent de fluide (9) et la première chambre (1) mesurée le long du canal d'aspiration capillaire (4) est d'au moins 1,2 mm, de préférence d'environ 1,3 mm.

9. Système selon l'une quelconque des revendications 1 à 8, **caractérisé en ce que** le canal d'aspiration capillaire (4) a une section transversale ronde à un diamètre de 0,4 mm à 2,5 mm, plus préférablement de 1,5 mm à 1,9 mm, et le plus préférablement d'environ 1,8 mm.

10. Système selon l'une quelconque des revendications 1 à 9, **caractérisé en ce que** le canal d'évent (6) a une section transversale ronde à un diamètre de 0,3 mm à 2,5 mm, de préférence d'environ 0,5 mm.

11. Système selon l'une quelconque des revendications 1 à 10, **caractérisé en ce que** la première extrémité (7) du canal d'évent (6) est située dans une concavité dans la surface latérale (14) du système microfluidique.

12. Système selon l'une quelconque des revendications 1 à 11, **caractérisé en ce qu'**il est constitué de deux couches de plastique liées de façon durable, liées par ultrasons, thermiquement ou par laser, de préférence par ultrasons.

13. Système selon l'une quelconque des revendications 1 à 12, **caractérisé en ce qu'**il est fabriqué à partir d'une matière plastique transparente, choisie dans le groupe comprenant polystyrène, PMMA, COP, COC, de préférence en polystyrène.

14. Système selon l'une quelconque des revendications 1 à 13, **caractérisé en ce qu'**il a la forme d'une plaque, limitée par le bas par le plan inférieur (101), par le dessus par le plan supérieur (201), sensiblement parallèle au plan inférieur (101), et limitée du côté par la surface latérale (14) reliant les plans du plan inférieur (101) et du plan supérieur (201), tandis que le plan supérieur (201) comprend un trou d'accès (13) menant à la première chambre (1) et situé au-dessus de la première chambre (1), permettant d'aspirer le fluide à partir de la première chambre (1), trou d'accès étant étroitement fermé avant la première utilisation du système.

15. Système selon la revendication 14, **caractérisé en ce que** le trou d'accès (13) est scellé avec une feuille, de préférence à double couche, comprenant notamment une couche de polystyrène du côté de la première chambre (1) et une couche d'aluminium du côté opposé, où ladite feuille peut être perforée avec un dispositif de distribution, de préférence une pointe de pipette automatique ou une aiguille lors de la collection de l'échantillon de la première chambre (1).

16. Système selon l'une quelconque des revendications 1 à 15, **caractérisé en ce que** la surface latérale (14) a une forme spéciale, en particulier elle comprend des rainures, des profils, des cavités ou des pattes qui facilitent une fixation stable du système dans le rotor de la centrifugeuse.

17. Système selon l'une quelconque des revendications 1 à 16, **caractérisé en ce que** les éléments du système microfluidique sont placés de telle sorte dans ce système qu'après avoir placé le système dans le rotor de centrifugation pour la centrifugation, l'axe de rotation est situé du même côté du système que la première extrémité (7) du canal d'évent (6) et l'entrée (5) du canal d'aspiration (4).

18. Système selon l'une quelconque des revendications 1 à 17, **caractérisé en ce que** le canal d'évent (6) a des parois transparentes qui sont visibles de l'extérieur du système et peuvent être utilisées comme marqueur de remplissage correct du système avec du sang.

19. Système selon l'une quelconque des revendications 1 à 18, **caractérisé en ce qu'**il comprend des anticoagulants, de préférence sous la forme d'un revêtement de la surface interne du canal d'aspiration (4) avec un agent anticoagulant, de préférence l'héparine.

20. Système selon l'une quelconque des revendications 1 à 19, **caractérisé en ce qu'**il comprend un bouchon (12), notamment en silicone, fixé sur le système microfluidique.
